(19) Europäisches Patentamt
European Patent Office
Office européen des brevets

(11) Numéro de publication: **0 362 006**
**A1**

(12) # DEMANDE DE BREVET EUROPEEN

(21) Numéro de dépôt: **89402479.3**

(51) Int. Cl.[5]: **C07D 215/22 , A61K 31/47**

(22) Date de dépôt: **12.09.89**

Revendications pour les Etats contractants suivants: ES + GR.

(30) Priorité: **13.09.88 FR 8811903**

(43) Date de publication de la demande:
**04.04.90 Bulletin 90/14**

(84) Etats contractants désignés:
**AT BE CH DE ES FR GB GR IT LI LU NL SE**

(71) Demandeur: **RHONE-POULENC SANTE**
**20, avenue Raymond Aron**
**F-92160 Antony(FR)**

(72) Inventeur: **Dubroeucq, Marie-Christine**
**13 Villa Malleville**
**F-95880 Enghien-Les-Bains(FR)**
Inventeur: **Paris, Jean-Marc**
**8 Rue des Acacias**
**F-77360 Vaires Sur Marne(FR)**
Inventeur: **Renault, Christian**
**61 Rue des Mallets**
**F-95150 Taverny(FR)**

(74) Mandataire: **Savina, Jacques et al**
**RHONE-POULENC SANTE Service Brevets 25, Quai Paul Doumer**
**F-92408 Courbevoie Cédex(FR)**

(54) **Dérivés de quinoléine, leurs procédés de préparation et les médicaments les contenant.**

(57) Composés de formule :

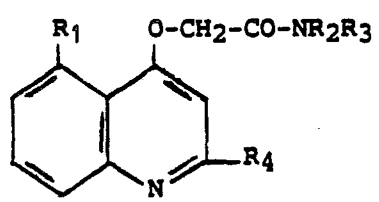

(I)

dans laquelle
- soit $R_1$ représente un radical trifluorométhyle, $R_2$ et $R_3$ forment ensemble avec l'atome d'azote auquel ils sont rattachés un radical morpholino et $R_4$ représente un radical méthyl-4 phényle, chloro-4 phényle, fluoro-2,3 ou 4 phényle, méthoxy-4 phényle, méthyl-4 fluoro-3 phényle, difluoro-2,4 phényle ou amino-4 phényle,
- soit $R_1$ représente un radical trifluorométhyle, $R_2$ et $R_3$ forment ensemble avec l'atome d'azote auquel ils sont rattachés un radical thiomorpholino ou $R_2$ représente un radical méthyle et $R_3$ représente un radical méthoxy-2 éthyle et $R_4$ représente un radical méthyl-4 phényle,
- soit $R_1$ représente un atome de chlore ou de fluor ou un radical méthoxy, $R_2$ et $R_3$ forment ensemble avec l'atome d'azote auquel ils sont rattachés un radical morpholino et $R_4$ représente un radical méthyl-4 phényle
leurs procédés de préparation et les médicaments les contenant.

EP 0 362 006 A1

## DERIVES DE QUINOLEINE, LEURS PROCEDES DE PREPARATION ET LES MEDICAMENTS LES CONTENANT

La présente invention concerne des dérivés de la quinoléine de formule :

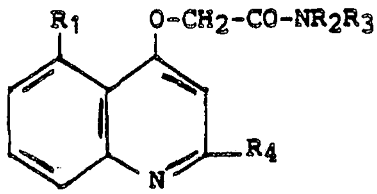

(I)

leurs procédés de préparation et les médicaments les contenant.

Dans la formule (I) :

- soit $R_1$ représente un radical trifluorométhyle, $R_2$ et $R_3$ forment ensemble avec l'atome d'azote auquel ils sont rattachés un radical morpholino et $R_4$ représente un radical méthyl-4 phényle, chloro-4 phényle, fluoro-2,3 ou 4 phényle, méthoxy-4 phényle, méthyl-4 fluoro-3 phényle, difluoro-2,4 phényle ou amino-4 phényle,
- soit $R_1$ représente un radical trifluorométhyle, $R_2$ et $R_3$ forment ensemble avec l'atome d'azote auquel ils sont rattachés un radical thiomorpholino, ou $R_2$ représente un radical méthyle et $R_3$ représente un radical méthoxy-2 éthyle et $R_4$ représente un radical méthyl-4 phényle,
- soit $R_1$ représente un atome de chlore ou de fluor ou un radical méthoxy, $R_2$ et $R_3$ forment ensemble avec l'atome d'azote auquel ils sont rattachés un radical morpholino et $R_4$ représente un radical méthyl-4 phényle.

Les composés de formule (I) à l'exception de celui pour lequel $R_4$ représente un radical amino-4 phényle peuvent être préparés par action d'une quinolone de formule :

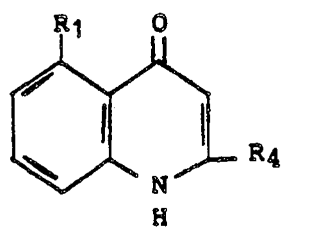

(II)

dans laquelle $R_1$ et $R_4$ ont les mêmes significations que dans la formule (I) excepté que $R_4$ ne peut pas représenter un radical amino-4 phényle sur un dérivé de formule :

$Hal - CH_2 - CO - NR_2R_3$ (III)

dans laquelle $R_2$ et $R_3$ ont les mêmes significations que dans la formule (I) et Hal représente un atome d'halogène (chlore ou brome de préférence).

Cette réaction s'effectue, de préférence, en présence d'un carbonate de métal alcalin tel que le carbonate de sodium ou de potassium, au sein d'un solvant organique tel qu'une cétone comme la butanone-2, à une température comprise entre 20 °C et la température d'ébullition du solvant.

Les quinolones de formule (II) peuvent être obtenues par cyclisation d'un dérivé de formule :

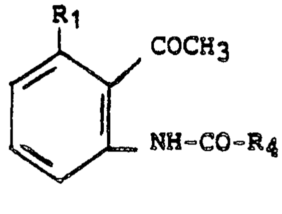

(IV)

dans laquelle $R_1$ et $R_4$ ont les mêmes significations que dans la formule (II).

Cette cyclisation s'effectue généralement dans un solvant organique tel que le benzène, le toluène ou

le xylène, en présence de tertbutylate de sodium, à la température d'ébullition du solvant.

Les composés de formule (IV) peuvent être obtenus par action d'un dérivé aminé de formule :

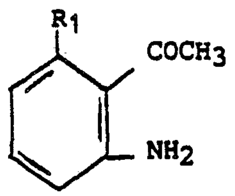

(V)

dans laquelle $R_1$ a les mêmes significations que dans la formule (I) ou un sel d'un tel dérivé avec un acide, sur un dérivé chloré de formule :

Cl - CO - $R_4$ (VI)

dans laquelle $R_4$ a les mêmes significations que dans la formule (II).

Cette réaction s'effectue, de préférence, au sein d'un solvant organique tel que le benzène, le toluène ou le xylène, en présence de pyridine, à une température voisine de 20° C.

Les dérivés aminés de formule (V) peuvent être obtenus par oxydation d'un dérivé de formule :

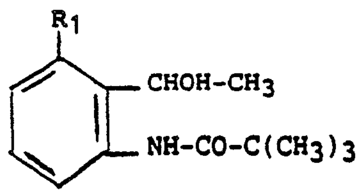

(VII)

dans laquelle $R_1$ a les mêmes significations que dans la formule (I) puis libération du groupe amine.

L'oxydation peut être effectuée au moyen d'anhydride chromique, au sein d'une cétone telle que l'acétone, à une température comprise entre -5° C et 0° C et la libération de l'amine au moyen d'un acide tel que l'acide chlorhydrique, au sein d'un alcool tel que le méthanol ou le butanol.

Les dérivés de formule (VII) peuvent être préparés par action d'acétaldéhyde sur un dérivé de formule :

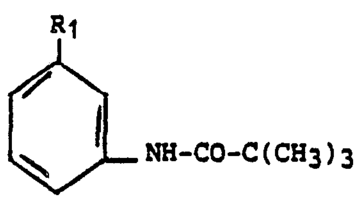

(VIII)

dans laquelle $R_1$ a les mêmes significations que dans la formule (I).

Cette réaction s'effectue au moyen de butyllithium dans un solvant organique tel que l'hexane à une température comprise entre -78° C et 20° C.

Les dérivés de formule (VIII) peuvent être obtenus par action de chlorure de pivaloyle sur un dérivé de formule :

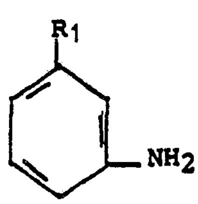

(IX)

dans laquelle $R_1$ a les mêmes significations que dans la formule (I).

Cette réaction s'effectue, de préférence, dans un solvant organique tel que le benzène ou le toluène, en

présence d'une trialkylamine ou de pyridine à une température comprise entre 0° C et 20° C.

Les dérivés de formule (III) pour lesquels $R_1$ représente un atome de chlore ou de fluor peuvent également être obtenus par action d'un dérivé de formule (IX) dans laquelle $R_1$ a les mêmes significations que précédemment sur le méthyl-4 benzoyl acétate d'éthyle.

Cette réaction s'effectue généralement au moyen d'acide polyphosphorique à une température comprise entre 100 et 170° C. Le produit est obtenu en mélange avec la fluoro ou chloro-7 (méthyl-4 phényl)-2 quinolone-4.

Ce mélange peut être utilisé tel quel pour la préparation du composé de formule (I).

Les composés de formule (I) à l'exception de celui pour lesquels $R_4$ représente un radical amino-4 phényle peuvent également être préparés par action d'un chlorure d'acide de formule :

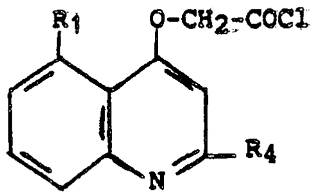

(X)

dans laquelle $R_1$ et $R_4$ ont les mêmes significations que dans la formule (I) excepté que $R_4$ ne peut pas représenter un radical amino-4 phényle, sur un amine de formule :

$HNR_2R_3$ (XI)

dans laquelle $R_2$ et $R_3$ ont les mêmes significations que dans la formule (I).

Cette réaction s'effectue, de préférence, au sein d'un solvant chloré tel que le chloroforme ou le chlorure de méthylène, en présence d'une amine tertiaire telle que la triéthylamine, à une température comprise entre 20° C et la température d'ébullition du solvant.

Les chlorures d'acide de formule (X) peuvent être obtenus par action d'un agent de chloruration tel que le chlorure de thionyle sur l'acide correspondant.

Cette réaction peut être réalisée au sein d'un solvant inerte tel que le chloroforme ou le toluène, de préférence à la température d'ébullition du solvant.

Les acides correspondants peuvent être préparés par hydrolyse des esters éthyliques correspondants.

Cette hydrolyse s'effectue généralement au moyen d'une solution aqueuse de soude à la température d'ébullition du milieu réactionnel.

Les esters éthyliques peuvent être obtenus par action d'une quinolone de formule (II) sur le bromoacétate d'éthyle.

Cette réaction s'effectue, de préférence, au sein d'une cétone comme la butanone-2, en présence d'un carbonate de métal alcalin tel que le carbonate de potassium ou de sodium, à la température d'ébullition du solvant.

Le composé de formule (I) pour lequel $R_4$ représente un radical amino-4 phényle peut être obtenu par réduction de la [((nitro-4 phényl)-2 trifluorométhyl-5 quinolyl-4)oxyacétyl]-4 morpholine.

Cette réaction s'effectue généralement au moyen de charbon palladié et d'acide chlohydrique, au sein d'un alcool tel que méthanol, éthanol, isopropanol ou un mélange de ces solvants.

La [((nitro-4 phényl)-2 trifluorométhyl-5 quinolyl-4)oxyacétyl]-4 morpholine peut être obtenue par les procédés décrits précédemment pour la prépration des composés de formule (I).

Les mélanges réactionnels obtenus par les divers procédés décrits précédemment sont traités suivant des méthodes classiques physiques (évaporation, extraction, distillation, cristallisation ou chromatographie).

Les composés de formule (I) présentent des propriétés pharmacologiques intéressantes révélatrices d'une activité anxiolytique, hypnotique, anticonvulsivante et antiépileptique.

Ces composés présentent une bonne affinité in vitro pour les sites récepteurs à benzodiazépine à des concentrations inférieures ou égales à 100 nM selon la technique décrite par J.C. BLANCHARD et L. JULOU, J. of Neurochemistry, 40, 601 (1983) inspirée des travaux de SQUIRES et BRAESTRUP, Nature, 266, 732 (1977).

Chez la souris, ils se sont montrés actifs à des doses inférieures ou égales à 25 mg/kg par voie orale vis-à-vis des convulsions induites par le pentétrazol selon une technique voisine de celle de EVERETT et RICHARDS, J. Pharmacol., 81, 402 (1944).

Les composés de formule (I) présentent une faible toxicité. Leur $DL_{50}$ est généralement supérieure à 300 mg/kg par voie orale chez la souris.

Sont particulièrement intéressants les composés de formule (I) dans laquelle $R_1$ représente un radical

trifluorométhyle, $R_2$ et $R_3$ forment ensemble avec l'atome d'azote auquel ils sont rattachés un cycle morpholino et $R_4$ représente un radical méthyl-4 phényle, chloro-4 phényle, fluoro-4 phényle, méthoxy-4 phényle, méthyl-4 fluoro-3 phényle ou difluoro-2,4 phényle.

Les exemples suivants, donnés à titre non limitatif, montrent comment l'invention peut être mise en pratique.

EXEMPLE 1

A une suspension agitée de 2,4 g de (méthyl-4 phényl)-2 trifluorométhyl-5 quinolone-4 et de 1,3 g de (chloro-2 acétyl)-4 morpholine dans 50 cm3 de butanone-2, on ajoute 2,2 g de carbonate de potassium anhydre. On chauffe au reflux 3 heures, refroidit à température ambiante (20 °C environ), élimine l'insoluble par filtration, le rince par de la butanone-2, rassemble les phases organiques et élimine le solvant sous pression réduite. Après recristallisation du résidu dans l'acétonitrile, on isole 2,6 g de [((méthyl-4 phényl)-2 trifluorométhyl-5 quinolyl-4) oxyacétyl]-4 morpho line fondant à 172 °C.

La (méthyl-4 phényl)-2 trifluorométhyl-5 quinolone-4 peut être préparée de la façon suivante : on chauffe au reflux pendant 1 heure 2,8 g de N-(acétyl-2 trifluorométhyl-3 phényl) méthyl-4 benzamide et 1,07 g de tertiobutylate de potassium dans 30 cm3 de toluène. On refroidit à température ambiante (20 °C environ), ajoute 0,55 cm3 d'acide acétique glacial et dilue par 30 cm3 d'eau et 15 cm3 d'éther éthylique. La suspension est agitée 30 minutes, le précipité est essoré, lavé 2 fois à l'éther éthylique puis à l'eau et enfin séché. Il présente un point de fusion supérieur à 260 °C.

Le N-(acétyl-2 trifluorométhyl-3 phényl) méthyl-4 benzamide peut être préparé de la façon suivante : on agite à température ambiante (20 °C environ) pendant 45 minutes, 2,39 g de chlorhydrate d'amino-2 trifluorométhyl-6 acétophénone, 1,45 cm3 de chlorure de méthyl-4 benzoyle et 1,7 cm3 de pyridine dans 20 cm3 de toluène anhydre. On ajoute ensuite 20 cm3 d'eau et 80 cm3 d'acétate d'éthyle. La phase aqueuse est décantée et la phase organique est lavée par trois fois 25 cm3 d'eau, séchée sur sulfate de magnésium et évaporée sous pression réduite. Le résidu obtenu est agité dans 50 cm3 d'éther de pétrole, filtré et lavé à l'éther de pétrole. Cette opération est réitérée deux fois. On isole ainsi 2,5 g de N-(acétyl-2 trifluorométhyl-3 phényl) méthyl-4 benzamide fondant à 171 °C.

Le chlorhydrate d'amino-2 trifluorométhyl-6 acétophénone peut être préparé de la façon suivante : on chauffe au reflux pendant 5 heures et 30 minutes, 67 g de N-(acétyl-2 trifluorométhyl-3 phényl) diméthyl-2,2 propionamide dans un mélange de 670 cm3 d'acide chlorhydrique concentré et de 670 cm3 d'éthanol. On refroidit à température ambiante (20 °C environ). Les solvants sont évaporés sous pression réduite, le résidu cristallisé est repris plusieurs fois au toluène en évaporant à chaque fois afin d'éliminer l'eau restante, lavé par de l'éther éthylique puis par de l'éther de pétrole 40 °-60 ° et enfin séché sous pression réduite. On obtient ainsi 50,4 g de chlorhydrate d'amino-2 trifluorométhyl-6 acétophénone fondant à 163 °C.

Le N-(acétyl-2 trifluorométhyl-3 phényl) diméthyl-2,2 pro pionamide peut être préparé de la façon suivante : à une solution, refroidie à -5 °C, de 36 g de N-((α-hydroxyéthyl)-2 trifluorométhyl-3 phényl) diméthyl-2,2 propionamide dans 1000 cm3 d'acétone on ajoute par portions, en 20 minutes, 37 g d'anhydride chromique. On agite 15 minutes à -5 °C puis on ajoute par portions 19 g supplémentaires d'anhydride chromique. On poursuit l'agitation encore 15 minutes à -5 °C puis introduit lentement à cette température 120 cm3 d'isopropanol afin de détruire l'excès d'anhydride chromique. On laisse remonter la température jusqu'à température ambiante (20 °C environ) et évapore le solvant sous pression réduite. Le résidu est repris par 700 cm3 d'eau et 1000 cm3 d'éther éthylique puis agité pendant une heure. On élimine par filtration un léger insoluble que l'on lave par de l'eau et de l'éther éthylique. Les phases aqueuses et organiques sont réunies, la phase aqueuse est décantée et lavée par deux fois 200 cm3 d'éther éthylique. Les phases organiques sont rassemblées, lavées par 2 fois 100 cm3 d'eau, 2 fois 100 cm3 d'une solution à 5% de bicarbonate de potassium et 2 fois 100 cm3 d'eau, séchées sur sulfate de magnésium puis évaporées sous pression réduite. On obtient ainsi 34,3 g de N-(acétyl-2 trifluorométhyl-3 phényl) diméthyl-2,2 propionamide fondant à 142 °C.

Le N-((α-hydroxyéthyl)-2 trifluorométhyl-3 phényl) diméthyl-2,2 propionamide peut être préparé de la façon suivante : à une solution de 7,7 g de N-(trifluorométhyl-3 phényl) diméthyl-2,2 propionamide dans 40 cm3 de tétrahydrofuranne sec placée sous atmosphère d'azote et refroidie à 10 °C, on ajoute, en 15 minutes, 45 cm3 d'une solution 1,37 M de n-butyllithium dans l'hexane et on agite 2 heures à cette température. On refroidit ensuite la solution à -78 °C et on introduit en une fois 17,5 cm3 d'acétaldéhyde refroidi à -78 °C. La température s'élève rapidement jusqu'à 12 °C puis redescend. Lorsque le milieu réactionnel est à -10 °C, on ajoute rapidement 150 cm3 d'eau. Après évaporation du tétrahydrofuranne sous pression réduite, on reprend la suspension par 200 cm3 d'éther éthylique et on agite jusqu'à dissolution. La

phase aqueuse est décantée, la phase organique est lavée par trois fois 20 cm3 d'eau, séchée sur sulfate de magnésium et évaporée sous pression réduite. Le résidu obtenu est repris par 100 cm3 d'éther de pétrole 40°-60° et délitté au reflux. La suspension blanche ainsi obtenue est refroidie, le précipité est essoré, empâté 2 fois à l'éther de pétrole, lavé avec de l'éther isopropylique préalablement refroidi à -70° C et enfin séché sous pression réduite. On obtient ainsi 6,2 g de N-((α-hydroxyéthyl)-2 trifluorométhyl-3 phényl) diméthyl-2,2 propionamide fondant à 172° C.

Le N-(trifluorométhyl-3 phényl) diméthyl-2,2 propionamide peut être préparé de la façon suivante : à une solution, sous atmosphère d'azote, de 130 g de trifluorométhyl-3 aniline dans 1300 cm3 de toluène, on ajoute 118 cm3 de triéthylamine. Après refroidissement à 10° C, on introduit, en 45 minutes, 105 cm3 de chlorure de pivaloyle et agite à une température voisine de 20° C pendant une heure. La suspension est reprise par 500 cm3 d'eau et 300 cm3 d'acétate d'éthyle. La phase organique est décantée et la phase aqueuse est lavée par 200 cm3 d'acétate d'éthyle. Les phases organiques sont rassemblées, lavées par 300 cm3 d'eau, 200 cm3 d'une solution normale d'acide chlorhydrique puis 200 cm3 d'eau et séchées sur sulfate de magnésium. Le solvant est éliminé sous pression réduite. Le résidu obtenu est repris par 3 fois 100 cm3 d'éther de pétrole 40°-60° puis séché. On obtient ainsi 183,4 g de N-(trifluorométhyl-3 phényl) diméthyl-2,2 propionamide fondant à 107° C.

EXEMPLE 2

On opère comme à l'exemple 1 mais en partant de 2,9 g de (chloro-4 phényl)-2 trifluorométhyl-5 quinolone-4, de 1,62 g de (chloro-2 acétyl)-4 morpholine et de 2,5 g de carbonate de potassium anhydre dans 60 cm3 de butanone-2. Après recristallisation dans l'acétonitrile, on isole 3,2 g de [((chloro-4 phényl)-2 trifluorométhyl-5 quinolyl-4) oxyacétyl]-4 morpholine fondant à 179° C.

La (chloro-4 phényl)-2 trifluorométhyl-5 quinolone-4 peut être préparée de la manière suivante : on opère comme à l'exemple 1 pour la préparation de la (méthyl-4 phényl)-2 trifluorométhyl-5 quinolone-4, à partir de N-(acétyl-2 trifluorométhyl-3 phényl) chloro-4 benzamide (3,5 g) et de tertiobutylate de potassium (1,24 g) dans 40 cm3 de toluène, en allongeant le temps de chauffage à 1 heure et 30 minutes. Elle présente un point de fusion supérieur à 260° C.

Le N-(acétyl-2 trifluorométhyl-3 phényl) chloro-4 benzamide peut être préparé de la manière suivante : on opère comme à l'exemple 1 pour la préparation du N-(acétyl-2 trifluorométhyl-3 phényl) méthyl-4 benzamide, à partir de chlorhydrate d'amino-2 trifluorométhyl-6 acétophénone (2,7 g), de chlorure de chloro-4 benzoyle. (1,58 cm3) et de 2 cm3 de pyridine dans 30 cm3 de toluène anhydre, en allongeant le temps de réaction à 2 heures. Il présente un point de fusion égal à 210° C.

EXEMPLE 3

On opère comme à l'exemple 1 mais en partant de 2,6 g de (fluoro-4 phényl)-2 trifluorométhyl-5 quinolone-4, de 1,53 g de (chloro-2 acétyl)-4 morpholine et de 2,35 g de carbonate de potassium anhydre dans 60 cm3 de butanone-2. Après recristallisation dans un mélange éther isopropylique-acétonitrile (37-10 en volumes), on isole 3,2 g de [((fluoro-4 phényl)-2 trifluorométhyl-5 quinolyl-4) oxyacétyl]-4 morpholine fondant à 164° C.

La (fluoro-4 phényl)-2 trifluorométhyl-5 quinolone-4 peut être préparée de la manière suivante : on opère comme à l'exemple 1 pour la préparation de la (méthyl-4 phényl)-2 trifluorométhyl-5 quinolone-4, à partir de N-(acétyl-2 trifluorométhyl-3 phényl) fluoro-4 benzamide (3 g) et de tertiobutylate de potassium (1,13 g) dans 40 cm3 de toluène, en allongeant à 2 heures le temps de réaction. Elle présente un point de fusion supérieur à 260° C.

Le N-(acétyl-2 trifluorométhyl-3 phényl) fluoro-4 benzamide peut être préparé de la manière suivante : on opère comme à l'exemple 1 pour la préparation du N-(acétyl-2 trifluorométhyl-3 phényl) méthyl-4 benzamide, à partir de chlorhydrate d'amino-2 trifluorométhyl-6 acétophénone (2,7 g), de chlorure de fluoro-4 benzoyle (1,49 cm3) et de 2 cm3 de pyridine dans 30 cm3 de toluène anhydre, en allongeant à 2 heures le temps de réaction. Il présente un point de fusion égal à 200° C.

EXEMPLE 4

On opère comme à l'exemple 1 mais en partant de 2,6 g de (fluoro-2 phényl)-2 trifluorométhyl-5

quinolone-4, de 1,53 g de (chloro-2 acétyl)-4 morpholine et de 2,35 g de carbonate de potassium anhydre dans 60 cm3 de butanone-2, en allongeant à 4 heures et 30 minutes le temps de réaction. Après recristallisation dans un mélange éther isopropylique-acétonitrile (37-16 en volumes), on isole 3,2 g de [(-(fluoro-2 phényl)-2 trifluorométhyl-5 quinolyl-4) oxyacétyl]-4 morpholine fondant à 172 °C.

La (fluoro-2 phényl)-2 trifluorométhyl-5 quinolone-4 peut être préparée de la manière suivante : on opère comme à l'exemple 1 pour la préparation de la (méthyl-4 phényl)-2 trifluorométhyl-5 quinolone-4, à partir de N-(acétyl-2 trifluorométhyl-3 phényl) fluoro-2 benzamide (3,2 g) et de tertiobutylate de potassium (1,2 g) dans 40 cm3 de toluène, en allongeant à 2 heures le temps de réaction. Elle présente un point de fusion supérieur à 260 °C.

Le N-(acétyl-2 trifluorométhyl-3 phényl) fluoro-2 benzamide peut être préparé de la manière suivante : on opère comme à l'exemple 1 pour la préparation du N-(acétyl-2 trifluorométhyl-3 phényl) méthyl-4 benzamide, à partir de chlorhydrate d'amino-2 trifluorométhyl-6 acétophénone (2,7 g), de chlorure de fluoro-2 benzoyle (1,5 cm3) et de 2 cm3 de pyridine dans 30 cm3 de toluène anhydre. Il présente un point de fusion égal à 100 °C.

EXEMPLE 5

On opère comme à l'exemple 1 mais en partant de 2,9 g de (méthoxy-4 phényl)-2 trifluorométhyl-5 quinolone-4, de 1,64 g de (chloro-2 acétyl)-4 morpholine et de 2,50 g de carbonate de potassium anhydre dans 60 cm3 de butanone-2. Après recristallisation dans l'acétonitrile, on isole 3,2 g de [((méthoxy-4 phényl)-2 trifluorométhyl-5 quinolyl-4) oxyacétyl]-4 morpholine fondant à 175 °C.

La (méthoxy-4 phényl)-2 trifluorométhyl-5 quinolone-4 peut être préparée de la manière suivante : on opère comme à l'exemple 1 pour la préparation de la (méthyl-4 phényl)-2 trifluorométhyl-5 quinolone-4, à partir de N-(acétyl-2 trifluorométhyl-3 phényl) méthoxy-4 benzamide (3,34 g) et de tertiobutylate de potassium (1,22 g) dans 40 cm3 de toluène, en allongeant à 6 heures le temps de réaction. Elle présente un point de fusion supérieur à 260 °C.

Le N-(acétyl-2 trifluorométhyl-3 phényl) méthoxy-4 benza mide peut être préparé de la manière suivante : on opère comme à l'exemple 1 pour la préparation du N-(acétyl-2 trifluorométhyl-3 phényl) méthyl-4 benzamide, à partir de chlorhydrate d'amino-2 trifluorométhyl-6 acétophénone (2,7 g), de chlorure de méthoxy-4 benzoyle (1,75 cm3) et de 2 cm3 de pyridine dans 30 cm3 de toluène anhydre, en allongeant à 2 heures le temps de réaction. Il présente un point de fusion égal à 171 °C.

EXEMPLE 6

On opère comme à l'exemple 1 mais en partant de 2,6 g de (méthyl-4 fluoro-3 phényl)-2 trifluorométhyl-5 quinolone-4, de 1,46 g de (chloro-2 acétyl)-4 morpholine et de 2,2 g de carbonate de potassium anhydre dans 60 cm3 de butanone-2. Après recristallisation dans un mélange éther isopropylique-acétonitrile (34-9 en volumes), on isole 2,7 g de [((méthyl-4 fluoro-3 phényl)-2 trifluorométhyl-5 quinolyl-4) oxyacétyl]-4 morpholine fondant à 169 °C.

La (méthyl-4 fluoro-3 phényl)-2 trifluorométhyl-5 quinolone-4 peut être préparée de la manière suivante : on opère comme à l'exemple 1 pour la préparation de la (méthyl-4 phényl)-2 trifluorométhyl-5 quinolone-4, à partir de N-(acétyl-2 trifluorométhyl-3 phényl) méthyl-4 fluoro-3 benzamide (2,9 g) et de tertiobutylate de potassium (1,06 g) dans 40 cm3 de toluène. Elle présente un point de fusion supérieur à 260 °C.

Le N-(acétyl-2 trifluorométhyl-3 phényl) méthyl-4 fluoro-3 benzamide peut être préparé de la manière suivante : on opère comme à l'exemple 1 pour la préparation du N-(acétyl-2 trifluorométhyl-3 phényl) méthyl-4 benzamide, à partir de chlorhydrate d'amino-2 trifluorométhyl-6 acétophénone (2,7 g), de chlorure de méthyl-4 fluoro-3 benzoyle (2,32 g) et de 2 cm3 de pyridine dans 30 cm3 de toluène anhydre. Il présente un point de fusion égal à 164 °C.

EXEMPLE 7

On opère comme à l'exemple 1 mais en partant de 1,8 g de (fluoro-3 phényl)-2 trifluorométhyl-5 quinolone-4, de 1,06 g de (chloro-2 acétyl)-4 morpholine et de 1,60 g de carbonate de potassium anhydre dans 36 cm3 de butanone-2. Le résidu (2,35 g) est dissous à chaud dans 25 cm3 d'acétate d'éthyle et la solution est filtrée à chaud. On ajoute 25 cm3 d'éther de pétrole 40 °-60 ° et le solide qui précipite est

séparé par filtration et séché. On isole 1,77 g de [((fluoro-3 phényl)-2 trifluorométhyl-5 quinolyl-4) oxyacétyl]-4 morpholine fondant à 157 °C.

La (fluoro-3 phényl)-2 trifluorométhyl-5 quinolone-4 peut être préparée de la manière suivante : on opère comme à l'exemple 1 pour la préparation de la (méthyl-4 phényl)-2 trifluorométhyl-5 quinolone-4, à partir de N-(acétyl-2 trifluorométhyl-3 phényl) fluoro-3 benzamide (2,56 g) et de tertiobutylate de potassium (0,97 g) dans 26 cm3 de toluène, en allongeant à 2 heures et 30 minutes le temps de réaction. Elle présente un point de fusion supérieur à 260 °C.

Le N-(acétyl-2 trifluorométhyl-3 phényl) fluoro-3 benzamide peut être préparé de la manière suivante : on opère comme à l'exemple 1 pour la préparation du N-(acétyl-2 trifluorométhyl-3 phényl) méthyl-4 benzamide, à partir de chlorhydrate d'amino-2 trifluorométhyl-6 acétophénone (2,3 g), de chlorure de fluoro-3 benzoyle (1,68 g) et de 1,7 cm3 de pyridine dans 26 cm3 de toluène anhydre, en allongeant à 2 heures le temps de réaction. Il présente un point de fusion égal à 150 °C.

## EXEMPLE 8

A une suspension agitée de 10 g d'un mélange de chloro-5 (méthyl-4 phényl)-2 quinolone-4 et de chloro-7 (méthyl-4 phényl)-2 quinolone-4 et de 10,2 g de carbonate de potassium anhydre dans 250 cm3 de butanone-2, on ajoute 7,9 g de (bromo-2 acétyl)-4 morpholine dans 50 cm3 de butanone-2. On chauffe au reflux 15 heures, refroidit à température ambiante (20 °C environ), élimine l'insoluble par filtration et évapore la butanone-2 sous pression réduite. Le résidu est repris par 200 cm3 d'eau et la phase aqueuse est extraite par 3 fois 100 cm3 de chlorure de méthylène. La phase organique est décantée, séchée et évaporée sous pression réduite. Après deux chromatographies du résidu sur du gel de silice, la première au moyen d'un mélange chloroforme-acétate d'éthyle (70-30 en volumes), la seconde en utilisant comme éluant un mélange chloroforme-acétate d'éthyle (80-20 en volumes), le solide obtenu est recristallisé dans l'acétate d'éthyle. On obtient ainsi 2 g de [(chloro-5 (méthyl-4 phényl)-2 quinolyl-4) oxyacétyl]-4 morpholine fondant à 170 °C.

Le mélange de chloro-5 (méthyl-4 phényl)-2 quinolone-4 et de chloro-7 (méthyl-4 phényl)-2 quinolone-4 peut être obtenu de la façon suivante : on chauffe à 150 °C pendant 20 minutes, sous agitation, 13 g de chloro-3 aniline et 41,2 g de méthyl-4 benzoylacétate d'éthyle dans 40 g d'acide polyphosphorique. On refroidit ensuite à 90 °C, ajoute 10 cm3 d'acide chlorhydrique concentré puis 100 cm3 d'eau. Le précipité est essoré, lavé par 3 fois 100 cm3 d'eau, 2 fois 50 cm3 d'éther éthylique et 2 fois 50 cm3 d'acétone. On obtient ainsi 30 g d'un mélange de chloro-5 (méthyl-4 phényl)-2 quinolone-4 et de chloro-7 (méthyl-4 phényl)-2 quinolone utilisé tel quel dans les synthèses ultérieures.

## EXEMPLE 9

A une suspension agitée de 2,19 g de (méthyl-4 phényl)-2 méthoxy-5 quinolone-4 et de 1,62 g de (chloro-2 acétyl)-4 morpholine dans 76 cm3 de butanone-2, on ajoute 2,28 g de carbonate de potassium anhydre. On chauffe au reflux 4 heures et 20 minutes, refroidit à température ambiante (20 °C environ) et évapore le solvant sous pression réduite. Le résidu est repris par de l'eau et de l'acétate d'éthyle, la phase organique est décantée, séchée sur sulfate de magnésium et évaporée sous pression réduite. Le résidu obtenu est chromatographié sur du gel de silice en utilisant un mélange chloroforme-acétone (95-5 en volumes). Le solide recueilli est agité dans 50 cm3 d'éther de pétrole 40 °-60 °. On isole ainsi 2,3 g de [((méthyl-4 phényl)-2 méthoxy-5 quinolyl-4) oxyacétyl]-4 morpholine fondant à 132 °C.

La (méthyl-4 phényl)-2 méthoxy-5 quinolone-4 peut être préparée de la façon suivante : on chauffe au reflux pendant 1 heure, 2,27 g de N-(acétyl-2 méthoxy-3 phényl) méthyl-4 benzamide et 1,36 g de tertiobutylate de potassium dans 23 cm3 de toluène. On refroidit à température ambiante (20 °C environ), ajoute 1,5 cm3 d'acide acétique glacial et dilue par 25 cm3 d'eau. La suspension est agitée 15 minutes, le précipité est essoré, lavé à l'eau, au toluène, à l'éther de pétrole 40 °-60 ° puis séché. Il présente un point de fusion égal à 207 °C.

Le N-(acétyl-2 méthoxy-3 phényl) méthyl-4 benzamide peut être préparé de la façon suivante : on agite à température ambiante (20 °C environ) pendant 1 heure et 20 minutes, 4 g d'amino-2 méthoxy-6 acétophénone, 3,5 cm3 de chlorure de méthyl-4 benzoyle et 2,15 cm3 de pyridine dans 40 cm3 de toluène anhydre. On ajoute ensuite 40 cm3 d'eau et 60 cm3 de toluène. La phase aqueuse est décantée ; la phase organique est lavée par trois fois 25 cm3 d'eau, séchée sur sulfate de magnésium et évaporée sous pression réduite. Le résidu obtenu est agité dans 75 cm3 d'éther de pétrole 40 °-60 ° et 25 cm3 d'acétate

d'éthyle, filtré et lavé par de l'éther de pétrole 40°-60°. On isole ainsi 2,44 g de N-(acétyl-2 méthoxy-3 phényl) méthyl-4 benzamide fondant vers 48° C.

L'amino-2 méthoxy-6 acétophénone peut être préparé de la façon suivante : on chauffe au reflux 4 heures et 40 minutes, 18 g de N-(acétyl-2 méthoxy-3 phényl)diméthyl-2,2 propionamide dans un mélange de 90 cm3 d'acide chlorhydrique concentré et de 90 cm3 d'éthanol. On refroidit à température ambiante (20° C environ), ajoute 100 cm3 d'acétate d'éthyle et 250 cm3 d'une solution saturée de carbonate de potassium. La phase aqueuse est extraite par de l'acétate d'éthyle et la phase organique est lavée à l'eau. Les phases organiques sont rassemblées, séchées sur sulfate de magnésium et évaporées sous pression réduite. L'huile obtenue est utilisée telle quelle dans les synthèses ultérieures.

Le N-(acétyl-2 méthoxy-3 phényl) diméthyl-2,2 propionamide peut être préparé de la façon suivante : à une solution refroidie à 0° C de 35 g de N-((α-hydroxyéthyl)-2 méthoxy-3 phényl) diméthyl-2,2 propionamide dans 525 cm3 d'acétone, on ajoute par portions, en 40 minutes, 41 g d'anhydride chromique. On agite 1 heure et 5 minutes à 0° C puis on ajoute par portions 21 g supplémentaires d'anhydride chromique. On poursuit l'agitation encore 1 heure et 45 minutes à 0° C puis introduit lentement à cette température 160 cm3 d'isopropanol afin de détruire l'excès d'anhydride chromique. On laisse remonter la température jusqu'à température ambiante (20° C environ) et évapore le solvant sous pression réduite. Le résidu est repris par 500 cm3 d'acétate d'éthyle et agité à température ambian te. Le solide est éliminé par filtration et lavé par 350 cm3 d'acétate d'éthyle. La phase organique est concentrée sous pression réduite et le résidu chromatographié sur du gel de silice au moyen du mélange chlorure de méthylène-acétone (99-1 en volumes). On isole ainsi 7,83 g de N-(acétyl-2 méthoxy-3 phényl) diméthyl-2,2 propionamide fondant en dessous de 48° C.

Le N-((α-hydroxyéthyl)-2 méthoxy-3 phényl) diméthyl-2,2 propionamide peut être préparé de la façon suivante : à une solution de 10 g de N-(méthoxy-3 phényl) diméthyl-2,2 propionamide dans 150 cm3 de tétrahydrofuranne sec placée sous atmosphère d'azote et refroidie à 0° C, on ajoute, en 25 minutes, 90,5 cm3 d'une solution 1,37 M de n-butyllithium dans l'hexane et on agite 2 heures à cette température. On refroidit ensuite la solution à -78° C et on introduit en une fois 27 cm3 d'acétaldéhyde refroidi à -78° C. La température s'élève rapidement jusqu'à 4° C puis redescend. Lorsque la température est redescendue à -10° C, on ajoute rapidement 57 cm3 d'eau et 200 cm3 d'éther éthylique. Après séparation des phases par décantation, on extrait la phase aqueuse à l'éther éthylique, réunit les phases organiques et les évapore. On ajoute au résidu obtenu 100 cm3 d'éther isopropylique, triture le résidu qui cristallise, ajoute ensuite 100 cm3 d'éther de pétrole 40°-60° et agite 15 minutes. Ce traitement est réitéré avec 50 cm3 d'éther de pétrole 40°-60° et 50 cm3 d'éther isopropylique. Après filtration et séchage, on obtient 9,65 g de N-((α-hydroxyéthyl)-2 méthoxy-3 phényl) diméthyl-2,2 propionamide fondant à 159° C.

Le N-(méthoxy-3 phényl) diméthyl-2,2 propionamide peut être préparé de la manière suivante : à une solution agitée de 110,7 g de méthoxy-3 aniline dans 1100 cm3 de toluène, on ajoute 79,7 cm3 de pyridine puis, lentement, 120,5 cm3 de chlorure de pivaloyle. On agite 1 heure et 15 minutes à une température voisine de 20° C puis ajoute 500 cm3 d'eau. Le précipité est essoré, lavé par de l'eau et du toluène et séché en étuve à 70° C sous pression réduite en présence de pastilles d'hydroxyde de sodium. On obtient ainsi 137,4 g de N-(méthoxy-3 phényl) diméthyl-2,2 propionamide fondant à 126° C.

EXEMPLE 10

A une suspension agitée de 3 g d'acide (méthyl-4 phényl)-2 trifluorométhyl-5 quinolyl-4 oxyacétique dans 90 cm3 de chloroforme, on ajoute lentement 1,8 cm3 de chlorure de thionyle. On chauffe 3 heures au reflux, évapore le solvant ainsi que le chlorure de thionyle sous pression réduite puis reprend deux fois au chloroforme en évaporant à chaque fois afin d'éliminer les traces de chlorure de thionyle. Le résidu obtenu est mis en suspension dans 90 cm3 de chloroforme, la solution est refroidie vers 10° C et on introduit 2,2 cm3 de triéthylamine. Toujours à cette température, on ajoute une solution de 0,8 cm3 de thiomorpholine dans 10 cm3 de chloroforme. On agite 15 heures à température ambiante, évapore le solvant sous pression réduite, reprend le résidu par 100 cm3 d'acétate d'éthyle et 60 cm3 d'eau. La phase organique est décantée, lavée par 50 cm3 d'eau, deux fois 20 cm3 d'une solution décinormale d'hydroxyde de sodium et enfin par 20 cm3 d'eau puis séchée sur sulfate de magnésium. Après évaporation des solvants sous pression réduite, on isole un résidu (3,3 g) que l'on chromatographie sur du gel de silice en utilisant un mélange chlorure de méthylène-acétate d'éthyle (95-5 en volumes). Le résidu obtenu est agité dans 30 cm3 d'éther de pétrole 40°-60°. On obtient ainsi 1,9 g de [((méthyl-4 phényl)-2 trifluorométhyl-5 quinolyl-4)-oxyacétyl]-4 thiomorpholine fondant à 154° C.

L'acide (méthyl-4 phényl)-2 trifluorométhyl-5 quinolyl-4 oxyacétique peut être préparé de la façon

suivante : on chauffe au reflux pendant 10 heures 28,6 g de (méthyl-4 phényl)-2 trifluorométhyl-5 quinolyl-4 oxyacétate d'éthyle et 115 cm3 d'une solution normale d'hydroxyde de sodium dans 700 cm3 d'eau. On refroidit à température ambiante, acidifie à pH 5 par addition de 7 cm3 d'acide acétique glacial et agite 1 heure et 30 minutes à 10°C. Le précipité est essoré, lavé à l'eau et séché. On obtient 25,6 g d'acide (méthyl-4 phényl)-2 trifluorométhyl-5 quinolyl-4 oxyacétique [Rf = 0,22 ; chromatographie sur plaque de gel de silice ; éluant ; acétate d'éthyle].

Le (méthyl-4 phényl)-2 trifluorométhyl-5 quinolyl-4 oxyacétate d'éthyle peut être préparé de la façon suivante : on chauffe 3 heures et 30 minutes au reflux 24,5 g de (méthyl-4 phényl)-2 trifluorométhyl-5 quinolone-4, 9,9 cm3 de bromoacétate d'éthyle et 23,4 g de carbonate de potassium anhydre dans 730 cm3 de butanone-2. On refroidit à température ambiante (20°C environ), essore l'insoluble sur fritté et le lave plusieurs fois à l'éther éthylique. Les phases organiques sont évaporées sous pression réduite, le résidu obtenu est repris par 250 cm3 d'éther de pétrole 40°-60° et chauffé au reflux. La suspension est ensuite refroidie à 10°C sous agitation rapide pendant 30 minutes. Le précipité est essoré, lavé avec un peu d'éther de pétrole 40°-60° et séché. On obtient ainsi 28,1 g de (méthyl-4 phényl)-2 trifluorométhyl-5 quinolyl-4 oxyacétate d'éthyle fondant à 134°C.

## EXEMPLE 11

A une suspension agitée de 3 g d'acide (méthyl-4 phényl)-2 trifluorométhyl-5 quinolyl-4 oxyacétique préparé selon l'exemple 8 dans 100 cm3 de chloroforme, on ajoute lentement 3 cm3 de chlorure de thionyle. On chauffe 3 heures au reflux, évapore le solvant ainsi que le chlorure de thionyle sous pression réduite, puis reprend deux fois au chloroforme en évaporant à chaque fois afin d'éliminer les traces de chlorure de thionyle. Le résidu obtenu est mis en suspension dans 100 cm3 de chloroforme, la solution est refroidie vers 10°C et on introduit 3 cm3 de triéthylamine. Toujours à cette température, on ajoute une solution de 1,25 g de N-(méthoxy-2 éthyl) N-méthyl amine dans 10 cm3 de chloroforme. On agite 15 heures à température ambiante (20°C environ), évapore le solvant sous pression réduite, reprend le résidu par 100 cm3 d'éther éthylique et 30 cm3 d'eau. La phase organique est décantée, lavée par 30 cm3 d'eau, deux fois 20 cm3 d'une solution décinormale d'hydroxyde de sodium et deux fois 20 cm3 d'eau puis séchée sur sulfate de magnésium. Après évaporation des solvants sous pression réduite, on isole un résidu (1,9 g), que l'on chromatographie sur du gel de silice en utilisant un mélange chlorure de méthylène-acétate d'éthyle (95-5 en volumes). Le résidu obtenu est agité dans 20 cm3 d'éther de pétrole 40°-60°. On obtient ainsi 1 g de N-méthyl N-(méthoxy-2 éthyl) ((méthyl-4 phényl)-2 trifluorométhyl-5 quinolyl-4) oxyacétamide fondant à 116°C.

## EXEMPLE 12

On opère comme à l'exemple 1 mais en partant de 2,3 g de (difluoro-2,4 phényl)-2 trifluorométhyl-5 quinolone-4, de 1,35 g de (chloro-2 acétyl)-4 morpholine et de 2,3 g de carbonate de potassium anhydre dans 70 cm3 de butanone-2, en allongeant à 5 heures le temps de réaction. Après recristallisation dans l'acétonitrile, on isole 2,3 g de [((difluoro-2,4 phényl)-2 trifluorométhyl-5 quinolyl-4) oxyacétyl]-4 morpholine fondant à 184°C.

La (difluoro-2,4 phényl)-2 trifluorométhyl-5 quinolone-4 peut être préparée de la manière suivante : on opère comme à l'exemple 1 pour la préparation de la (méthyl-4 phényl)-2 trifluorométhyl-5 quinolone-4, à partir de 3,4 g de N-(acétyl-2 trifluorométhyl-3 phényl) difluoro-2,4 benzamide et de 1,3 g de tertiobutylate de potassium dans 40 cm3 de toluène, en allongeant à 2 heures et 30 minutes le temps de réaction. Elle présente un point de fusion supérieur à 260°C.

Le N-(acétyl-2 trifluorométhyl-3 phényl) difluoro-2,4 benzamide peut être préparé de la manière suivante : on opère comme à l'exemple 1 pour la préparation du N-(acétyl-2 trifluorométhyl-3 phényl) méthyl-4 benzamide, à partir de 3 g de chlorhydrate d'amino-2 trifluorométhyl-6 acétophénone préparé selon l'exemple 1, de 2,29 g de chlorure de difluoro-2,4 benzoyle et de 2,2 cm3 de pyridine dans 35 cm3 de toluène anhydre. Ce composé présente un Rf égal à 0,61 (chromatographie sur plaque de gel de silice, éluant : cyclohexane-acétate d'éthyle (50-50 en volumes)).

## EXEMPLE 13

A une suspension agitée de 11,2 g d'un mélange de fluoro-5 (méthyl-4 phényl)-2 quinolone-4 et de fluoro-7 (méthyl-4 phényl)-2 quinolone-4 et de 11,3 g de carbonate de potassium anhydre dans 250 cm3 de butanone-2, on ajoute 7,3 g de (bromo-2 acétyl)-4 morpholine dans 50 cm3 de butanone-2. On chauffe au reflux 15 heures, refroidit à température ambiante (20°C environ), élimine l'insoluble par filtration, et évapore la butanone-2 sous pression réduite. Le résidu est repris par 200 cm3 d'eau et la phase aqueuse est extraite par 3 fois 100 cm3 de chlorure de méthylène. La phase organique est décantée, séchée et évaporée sous pression réduite. Après chromatographie du résidu sur du gel de silice au moyen d'un mélange cyclohexane-acétate d'éthyle (70-30 en volumes) comme éluant, le solide obtenu est trituré dans 100 cm3 d'éther de pétrole 40°C-60°C. On obtient ainsi 2,4 g de [(fluoro-5 (méthyl-4 phényl)-2 quinolyl-4) oxyacétyl]-4 morpholine fondant à 142-4°C.

Le mélange de fluoro-5 (méthyl-4 phényl)-2 quinolone-4 et de fluoro-7 (méthyl-4 phényl)-2 quinolone-4 peut être obtenu de la façon suivante : On chauffe à 150°C pendant 30 minutes sous bonne agitation 7,3 g de fluoro-3 aniline et 26,7 g de méthyl-4 benzoyl acétate d'éthyle dans 27 g d'acide polyphosphorique. On refroidit ensuite à 90°C et ajoute 60 cm3 d'acide chlorhydrique normal. Le précipité est essoré, lavé par trois fois 60 cm3 d'eau, puis par trois fois 60 cm3 d'éther éthylique et enfin par deux fois 50 cm3 d'acétone. On obtient ainsi 22,9 g d'un mélange de fluoro-5 (méthyl-4 phényl)-2 quinolone-4 et de fluoro-7 (méthyl-4 phényl)-2 quinolone-4 que l'on utilise tel quel dans l'étape suivante.

## EXEMPLE 14

On hydrogène pendant trois heures trente minutes sous pression atmosphèrique 6,6 g de [((nitro-4 phényl)-2 trifluorométhyl-5 quinolyl-4)oxyacétyl]-4 morpholine en solution dans 132 cm3 de méthanol en présence de 6,6 cm3 d'une solution 8N d'acide chlorhydrique gazeux dans l'isopropanol et de 0,66 g de palladium supporté sur charbon à 10 %. On ajoute ensuite 240 cm3 d'eau distillée, agite 10 minutes puis filtre le catalyseur. On ajoute 45 cm3 d'une solution normale aqueuse d'hydroxyde de sodium, filtre le précipité, le lave par trois fois 100 cm3 d'eau distillée et le sèche sous pression réduite. Le précipité est dissous à chaud dans un mélange cyclohexane - acétate d'éthyle ( 50-50 en volumes). La solution est filtrée et le filtrat est concentré sous pression réduite. Le résidu est repris dans l'éther de pétrole, filtré et séché. On obtient ainsi 3,16 g d'[((amino-4 phényl)-2 trifluorométhyl-5 quinolyl-4)oxyacétyl]-4 morpholine fondant à 174°C.

La [((nitro-4 phényl)-2 trifluorométhyl-5 quinolyl-4)oxyacétyl]-4 morpholine peut être préparée de la manière suivante : on opère comme à l'exemple 1 pour la préparation de la [((méthyl-4 phényl)-2 trifluorométhyl-5 quinolyl-4)oxyacétyl]-4 morpholine, à partir de 8g de ((nitro-4 phényl)-2 trifluorométhyl-5 quinolone-4, de 6,6 g de carbonate de potassium anhydre et de 4,3 g de( chloro-2 acétyl)-4 morpholine dans 160 cm3 de butanone-2. Après recristallisation dans la butanone-2 on obtient 6,2 g de [((nitro-4 phényl)-2 trifluorométhyl-5 quinolyl-4)oxyacétyl]-4 morpholine fondant à 222°C.

La (nitro-4 phényl)-2 trifluorométhyl-5 quinolone-4 peut être préparée de la façon suivante : on opère comme à l'exemple 1 pour la préparation de la (méthyl-4 phényl)-2 trifluorométhyl-5 quinolone-4, à partir de 10,6 g de N-(acétyl-2 trifluorométhyl-3 phényl) nitro-4 benzamide et de 3,7 g de tertiobutylate de potassium dans un mélange de 106 cm3 de toluène et de 10 cm3 de diméthylformamide. On obtient 8,15 g de (nitro-4 phényl)-2 trifluorométhyl-5 quinolone-4 fondant au-dessus de 260°C.

Le N-(acétyl-2 trifluorométhyl-3 phényl) nitro-4 benzamide peut être préparé de la façon suivante : on opère comme à l'exemple 1 pour la préparation du N-(acétyl-2 trifluorométhyl-3 phényl) méthyl-4 benzamide, à partir de 10 g de chlorhydrate d'amino-2 trifluorométhyl-6 acétophénone et de 10,1 g de chlorure de l'acide nitro-4 benzoïque dans un mélange de 100 cm3 de toluène et de 8,4 cm3 de pyridine. On obtient ainsi 10,8 g de N-(acétyl-2 trifluorométhyl-3 phényl) nitro-4 benzamide fondant à 245°C.

La présente invention concerne également les médicaments qui contiennent les composés de formule (I) à l'état pur ou sous forme de compositions dans lesquelles ils sont associés à un adjuvant, un diluant et/ou un enrobage compatible et pharmaceutiquement acceptable. Ces médicaments peuvent être employés par voie orale, rectale, parentérale ou percutanée.

Comme compositions solides pour administration orale, peuvent être utilisés des comprimés, des pilules, des poudres (généralement dans des capsules de gélatine) ou des granulés. Dans ces compositions, le produit actif selon l'invention est mélangé à un ou plusieurs diluants inertes, tels que saccharose, lactose, amidon, cellulose ou silice. Ces compositions peuvent également comprendre des substances autres que les diluants, par exemple un lubrifiant tel que le stéarate de magnésium ou le talc, un colorant, un enrobage (dragées) ou un vernis.

Comme compositions liquides pour administration orale, on peut utiliser des émulsions pharmaceutiquement acceptables, des solutions, des suspensions, des sirops et des élixirs contenant des diluants inertes tels que l'eau, l'éthanol, le glycérol, les huiles végétales ou l'huile de paraffine. Ces compositions peuvent également comprendre des substances autres que les diluants, par exemple des produits mouillants, édulcorants, aromatisants, épaississants ou stabilisants.

Les compositions selon l'invention pour administration parentérale peuvent être des solutions stériles aqueuses ou non aqueuses, des suspensions ou des émulsions. Comme solvant ou véhicule, on peut employer le propylèneglycol, un polyéthylèneglycol, des huiles végétales, en particulier l'huile d'olive, des esters organiques injectables, par exemple l'oléate d'éthyle. Ces compositions peuvent également contenir des adjuvants, en particulier des agents mouillants, isotonisants, émulsifiants, stabilisants et dispersants. La stérilisation peut se faire de plusieurs façons, par exemple à l'aide d'un filtre bactériologique, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui peuvent contenir, outre le produit actif, des excipients tels que le beurre de cacao, la suppo-cire, des glycérides semi-synthétiques ou des polyéthylèneglycols.

Les compositions pour administration percutanée sont les crèmes, pommades, lotions et liniments, dans lesquels le produit actif est associé à des excipients liquides ou pâteux, de préférence en association avec un véhicule favorisant la migration percutanée.

Les médicaments et compositions selon l'invention sont particulièrement utiles en thérapeutique humaine pour leur action anxiolytique, hypnotique, anticonvulsivante et antiépileptique.

En thérapeutique humaine, les doses dépendent de l'effet recherché et de la durée du traitement ; elles sont généralement comprises entre 10 et 500 mg par jour par voie orale pour un adulte avec des doses unitaires allant de 2 à 100 mg de substance active. D'une façon générale, le médecin déterminera la posologie appropriée en fonction de l'âge, du poids et de tous les autres facteurs propres au sujet à traiter.

Les exemples suivants illustrent des compositions selon l'invention :

EXEMPLE A

On prépare, selon la technique habituelle, des gélules dosées à 50 mg de produit actif ayant la composition suivante :

| | |
|---|---|
| - [((méthyl-4 phényl)-2 trifluorométhyl-5 quinolyl-4) oxyacétyl]-4 morpholine ... | 50 mg |
| - cellullose ... | 18 mg |
| - lactose... | 55 mg |
| - silice colloïdale ... | 1 mg |
| - carboxyméthylamidon sodique ... | 10 mg |
| - talc ... | 10 mg |
| - stéarate de magnésium ... | 1 mg |

EXEMPLE B

On prépare, selon la technique habituelle, des comprimés dosés à 50 mg de produit actif ayant la composition suivante :

| | |
|---|---|
| - [((fluoro-4 phényl)-2 trifluorométhyl-5 quinolyl-4) oxyacétyl]-4 morpholine ... | 50 mg |
| - lactose... | 104 mg |
| - cellulose ... | 40 mg |
| - polyvidone ... | 10 mg |
| - carboxyméthylamidon sodique ... | 22 mg |
| - talc ... | 10 mg |
| - stéarate de magnésium ... | 2 mg |
| - silice colloïdale ... | 2 mg |
| - mélange d'hydroxyméthylcellulose, glycérine, oxyde de titane (72-3,5-24,5) ... q. s. p. | 1 comprimé pelliculé terminé à 245 mg |

EXEMPLE C

On prépare une solution injectable contenant 10 mg de produit actif ayant la composition suivante :

| | |
|---|---|
| - [((méthyl-4 fluoro-3 phényl)-2 trifluorométhyl-5 quinolyl-4) oxyacétyl]-4 morpholine ... | 10 mg |
| - acide benzoïque ... | 80 mg |
| - alcool benzylique ... | 0,06 cm3 |
| - benzoate de sodium ... | 80 mg |
| - éthanol à 95 % ... | 0,4 cm3 |
| - hydroxyde de sodium ... | 24 mg |
| - propylène glycol ... | 1,6 cm3 |
| - eau ... q. s. p. | 4 cm3 |

## Revendications

1. Composés de formule :

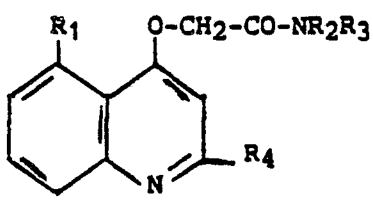

(I)

dans laquelle
- soit $R_1$ représente un radical trifluorométhyle, $R_2$ et $R_3$ forment ensemble avec l'atome d'azote auquel ils sont rattachés un radical morpholino et $R_4$ représente un radical méthyl-4 phényle, chloro-4 phényle, fluoro-2,3 ou 4 phényle, méthoxy-4 phényle, méthyl-4 fluoro-3 phényle, difluoro-2,4 phényle ou amino-4 phényle,
- soit $R_1$ représente un radical trifluorométhyle, $R_2$ et $R_3$ forment ensemble avec l'atome d'azote auquel ils sont rattachés un radical thiomorpholino ou $R_2$ représente un radical méthyle et $R_3$ représente un radical méthoxy-2 éthyle et $R_4$ représente un radical méthyl-4 phényle,
- soit $R_1$ représente un atome de chlore ou de fluor ou un radical méthoxy, $R_2$ et $R_3$ forment ensemble avec l'atome d'azote auquel ils sont rattachés un radical morpholino et $R_4$ représente un radical méthyl-4 phényle.

2. Composés de formule (I) selon la revendication 1 pour lesquels $R_1$ représente un radical trifluorométhyle, $R_2$ et $R_3$ forment ensemble avec l'atome d'azote auquel ils sont rattachés un cycle morpholino et $R_4$

représente un radical méthyl-4 phényle, chloro-4 phényle, fluoro-4 phényle, méthoxy-4 phényle, méthyl-4 fluoro-3 phényle ou difluoro-2,4 phényle.

3. La [((méthyl-4 phényl)-2 trifluorométhyl-5 quinolyl-4) oxyacétyl]-4 morpholine.

4. Procédé de préparation des composés de formule (I) selon la revendication 1 à l'exception de celui pour lequel $R_4$ représente un radical amino-4 phényle caractérisé en ce que l'on fait réagir une quinolone de formule :

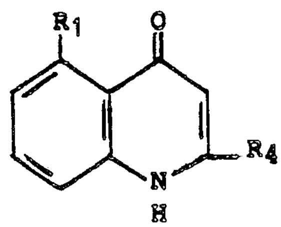

(II)

dans laquelle $R_1$ et $R_4$ ont les mêmes significations que dans la revendication 1 excepté que $R_4$ ne peut pas représenter un radical amino-4 phényle, sur un dérivé de formule : Hal - $CH_2$ - CO - $NR_2R_3$ (III)
dans laquelle $R_2$ et $R_3$ ont les mêmes significations que dans la revendication 1 et Hal représente un atome d'halogène et isole le produit.

5. Procédé de préparation des composés de formule (I) selon la revendication 1 à l'exception de celui pour lequel $R_4$ représente un radical amino-4 phényle caractérisé en ce que l'on fait réagir un composé de formule :

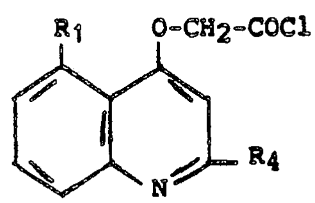

(X)

sur un amine de formule :
$HNR_2R_3$ (XI)
dans lesquelles $R_1$, $R_2$, $R_3$ et $R_4$ ont les mêmes significations que dans la revendication 1 excepté que $R_4$ ne peut pas représenter un radical amino-4 phényle.

6. Procédé de préparation du composé de formule (I) selon la revendication 1 pour lequel $R_4$ représente un radical amino-4 phényle caractérisé en ce que l'on réduit la [((nitro-4 phényl)-2 trifluorométhyl-5 quinolyl-4) oxyacétyl]-4 morpholine et isole de produit.

7. Médicaments caractérisés en ce qu'ils contiennent comme principe actif au moins un composé selon la revendication 1.

Revendications pour les Etats contractants suivants: GR,ES

1. Procédé de préparation des composés de formule :

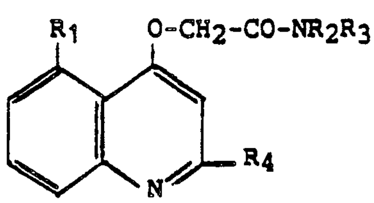

(I)

dans laquelle
- soit $R_1$ représente un radical trifluorométhyle, $R_2$ et $R_3$ forment ensemble avec l'atome d'azote auquel ils sont rattachés un radical morpholino et $R_4$ représente un radical méthyl-4 phényle, chloro-4 phényle, fluoro-2,3 ou 4 phényle, méthoxy-4 phényle, méthyl-4 fluoro-3 phényle, difluoro-2,4 phényle ou amino-4 phényle,
- soit $R_1$ représente un radical trifluorométhyle, $R_2$ et $R_3$ forment ensemble avec l'atome d'azote auquel ils sont rattachés un radical thiomorpholino ou $R_2$ représente un radical méthyle et $R_3$ représente un radical méthoxy-2 éthyle et $R_4$ représente un radical méthyl-4 phényle,
- soit $R_1$ représente un atome de chlore ou de fluor ou un radical méthoxy, $R_2$ et $R_3$ forment ensemble avec l'atome d'azote auquel ils sont rattachés un radical morpholino et $R_4$ représente un radical méthyl-4 phényle caractérisé en ce que

A - Pour la préparation des composés de formule (I) à l'exception de celui pour lequel $R_4$ représente un radical amino-4 phényle, on fait réagir une quinolone de formule :

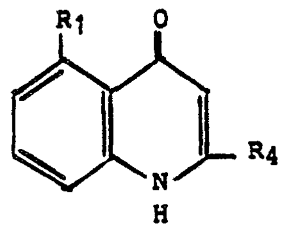

(II)

dans laquelle $R_1$ et $R_4$ ont les mêmes significations mentionnées précédemment excepté que $R_4$ ne peut pas être un radical amino-4 phényle sur un dérivé de formule :

$Hal - CH_2 - CO - NR_2R_3$ (III)

dans laquelle $R_2$ et $R_3$ ont les mêmes significations que ci-dessus et Hal représente un atome d'halogène puis isole le produit,

B - Pour la préparation des composés de formule (I) à l'exception de celui pour lequel $R_4$ représente un radical amino-4 phényle, on fait réagir un composé de formule :

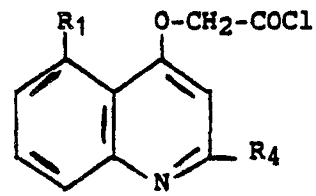

(X)

sur un amine de formule :

$HNR_2R_3$ (XI)

dans lesquelles $R_1$, $R_2$, $R_3$ et $R_4$ ont les mêmes significations que ci-dessus excepté que $R_4$ ne peut pas être un radical amino-4 phényle puis isole le produit.

C - Pour la préparation du composé de formule (I) pour lequel $R_4$ représente un radical amino-4 phényle on réduit la [((nitro-4 phényl)-2 trifluorométhyl-5 quinolyl-4) oxyacétyl]-4 morpholine puis isole de produit.

2. Procédé selon la revendication 1 pour la préparation des composés de formule (I) pour lesquels $R_1$ représente un radical trifluorométhyle, $R_2$ et $R_3$ forment ensemble avec l'atome d'azote auquel ils sont rattachés un cycle morpholino et $R_4$ représente un radical méthyl-4 phényle, chloro-4 phényle, fluoro-4 phényle, méthoxy-4 phényle, méthyl-4 fluoro-3 phényle ou difluoro-2,4 phényle.

3. Procédé selon la revendication 1 pour la préparation de la [((méthyl-4 phényl)-2 trifluorométhyl-5 quinolyl-4) oxyacétyl]-4 morpholine.

Office européen des brevets

# RAPPORT DE RECHERCHE EUROPEENNE

Numero de la demande

EP 89 40 2479

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X,Y | EP-A-0 205 375 (RHONE-POULENC)<br>* Page 7, ligne 34 - page 9, ligne 4; page 22, lignes 5-18; revendications 1,9 *<br>--- | 1-3,7 | C 07 D 215/22<br>A 61 K 31/47 |
| Y | EP-A-0 210 084 (RHONE-POULENC)<br>* Page 72, ligne 29 - page 73, ligne 4; revendications 1,10 *<br>----- | 1-3,7 | |
| | | | **DOMAINES TECHNIQUES RECHERCHES (Int. Cl.5)** |
| | | | C 07 D 215/00<br>A 61 K 31/00 |

Le présent rapport a été établi pour toutes les revendications

| Lieu de la recherche | Date d'achèvement de la recherche | Examinateur |
|---|---|---|
| LA HAYE | 15-12-1989 | BRENNAN J. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)